Europäisches Patentamt

European Patent Office

Office européen des brevets

(11) Veröffentlichungsnummer: **0 173 916**
**B1**

(12) **EUROPÄISCHE PATENTSCHRIFT**

(45) Veröffentlichungstag der Patentschrift:
08.02.89

(21) Anmeldenummer: 85110510.6

(22) Anmeldetag: 21.08.85

(51) Int. Cl.⁴: **C 12 Q 1/56** // G01N33/86

(54) Verfahren zur Bestimmung von Fibrinmonomer in Plasma.

(30) Priorität: 22.08.84 DE 3430906

(43) Veröffentlichungstag der Anmeldung:
12.03.86 Patentblatt 86/11

(45) Bekanntmachung des Hinweises auf die Patenterteilung:
08.02.89 Patentblatt 89/6

(84) Benannte Vertragsstaaten:
AT BE CH DE FR GB IT LI LU NL SE

(56) Entgegenhaltungen:
EP-A- 0 011 463
EP-A- 0 039 885
EP-A- 0 094 720
EP-A- 0 141 263
WO-A-81/00578

(73) Patentinhaber: BOEHRINGER MANNHEIM GMBH,
Sandhofer Strasse 112-132, D-6800 Mannheim-Waldhof
(DE)

(72) Erfinder: Bartl, Knut, Dr. rer. nat., Am Westend 6,
D-8121 Wilzhofen (DE)
Erfinder: Lill, Helmut, Dr. rer. nat., Zugspitzstrasse 24,
D-8121 Wielenbach (DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)

## Beschreibung

Die Erfindung betrifft ein Verfahren zur Bestimmung von Fibrinmonomer im Plasma und ein hierfür geeignetes Reagenz.

Die Erkennung einer Thrombosebereitschaft spielt in der klinischen Diagnostik, beispielsweise vor oder nach Operationen, eine wichtige Rolle. Ein Testparameter zur Erkennung einer derartigen Thrombosebereitschaft ist die im Serum vorhandene Menge an Fibrinmonomer. Ein über den Normalwert erhöhter Gehalt an Fibrinmonomer im Plasma zeigt einen latent ablaufenden Gerinnungsvorgang an. Zur Bestimmung von Fibrinmonomer im Plasma sind bereits verschiedene Methoden bekannt, die jedoch alle Nachteile aufweisen. So ist es bekannt, Fibrinmonomer durch Fällung mit Ethanol oder Protaminsulfat zu bestimmen. Diese Fällungsmethoden beinhalten die Gefahr einer unspezifischen Präzipitation. Sie sind auch nicht ausreichend empfindlich und geben daher nur qualitative Hinweise.

Weiter sind bekannt Bestimmungsmethoden durch Gelchromatographie oder Affinitätschromatographie an Fibrinagarose. Diese Verfahren sind nicht nur ungenügend empfindlich, sondern auch zu aufwendig, um für die Routinediagnose praktikabel zu sein.

Weiter ist bekannt ein Agglutinationsverfahren mit Erythrozyten, welche vorweg mit Fibrinmonomer beladen wurden. Auch dieses Verfahren weist eine unbefriedigende Empfindlichkeit auf.

Weiter ist ein radioaktives Verfahren bekannt, bei welchem $^{14}$C-Glycin aus entsprechend markierten Glycinestern mit Hilfe von Faktor XIII in Fibrin eingebaut wird. Dieses Verfahren ist zwar ausreichend empfindlich, erfordert aber die üblichen aufwendigen Massnahmen und Vorrichtungen beim Umgang mit radioaktiven Substanzen.

Schliesslich ist der EP-A 139885 der gleichen Anmelderin ein turbidimetrisches Verfahren zur Bestimmung von Fibrinmonomer in Plasma offenbart, bei welchem hydrophobe Latexteilchen mit Probe und Puffer in einer Küvette gemischt und der Extinktionsanstieg als Folge der Aggregation der Latexteilchen gemessen wird. Das Verfahren ist zwar sehr empfindlich mit einer Nachweisgrenze von ca. 1,2 µg/ml und für die Routinediagnostik geeignet. Es beruht jedoch auf einer an sich unspezifischen Adsorption und kann daher Störungen unterliegen.

Der Erfindung liegt nunmehr die Aufgabe zugrunde, ein Verfahren der obengenannten Art zu schaffen, welches die erwähnten Nachteile der bekannten Verfahren nicht aufweist, auf spezifischer, biochemischer Grundlage beruht, sehr empfindlich ist und durch einfache Farbmessungen im sichtbaren Bereich durchgeführt werden kann.

Gelöst wird diese Aufgabe erfindungsgemäss durch ein Verfahren zur Bestimmung von Fibrinmonomer in Plasma, welches dadurch gekennzeichnet ist, dass man die Fibrinmonomer enthaltende, von Plasmininhibitoren befreite Plasmaprobe mit Gewebs-Plasminogenaktivator (EPA), Plasminogen und einem chromogenen Plasminsubstrat in gepufferter Lösung inkubiert und die gebildete Farbe als Mass für die Fibrinmonomermenge misst.

Das Verfahren der Erfindung beruht darauf, dass unter dem Einfluss von Thrombin aus Fibrinogen Fibrinmonomer unter Abspaltung von Fibrinopeptid A gebildet wird. Gebildetes Fibrinmonomer wiederum aktiviert EPA, EPA führt zur Plasminbildung aus Plasminogen und das gebildete Plasmin, welches an sich die Fibrinolysereaktion in Gang setzt, spaltet das angebotene chromogene Substrat. Das erfindungsgemässe Verfahren nutzt also die aktivierende Wirksamkeit von Fibrinmonomer in bezug auf EPA aus.

Für das erfindungsgemässe Verfahren muss die zu untersuchende Plasmaprobe erst von Plasmininhibitoren befreit werden. Hierfür stehen verschiedene Methoden zur Verfügung. Gemäss einer ersten bevorzugten Ausführungsform wird das zu untersuchende Plasma mit Säure versetzt unter Bildung eines Niederschlags, der abgetrennt wird, beispielsweise abzentrifugiert wird, und dann in Pufferlösung suspendiert in das Verfahren als «Plasmaprobe» eingesetzt wird. Als Säure kann an sich eine beliebige organische oder anorganische Säure verwendet werden, bevorzugt wird Essigsäure verwendet. Die zuzusetzende Menge liegt zweckmässig bei 0,1 bis 0,5 Vol.-%, bezogen auf Plasmavolumen, vorzugsweise etwa 0,2 Vol.-%. Die Fällung erfolgt vorzugsweise in mit Wasser verdünntem, zweckmässig 1:5 bis 1:15 mit Wasser verdünnter Lösung. Nach dem Zusatz der Säure lässt man einige Zeit in der Kälte stehen und zentrifugiert anschliessend den gebildeten Niederschlag ab.

Eine alternative bevorzugte Methode zur Entfernung der Plasmininhibitoren besteht in der Inkubation des zu untersuchenden Plasmas mit einem Polystyrolkörper. Hierbei bindet sich das Fibrinmonomer an die Polystyroloberfläche. Nach einiger Zeit, beispielsweise 0,25 bis 24 Stunden, kann die Flüssigkeit abgegossen, vorsichtig mit Wasser nachgewaschen und anschliessend der mit dem Fibrinmonomer bedeckte Polystyrolkörper quasi als Probe eingesetzt werden. Durch längere Verweilzeiten kann die Empfindlichkeit des Tests erhöht werden. Als Polystyrolformkörper kommen beispielsweise Polystyrolröhrchen, Polystyrolkugeln, -flocken und dergleichen in Betracht. Bei Verwendung von Polystyrolröhrchen, beispielsweise in Form von Vertiefungen in Mikrotiterplatten, lässt sich das erfindungsgemässe Verfahren einfach so durchführen, dass man das Röhrchen gleich als Messküvette verwendet, indem man die anderen, für die Bestimmung erforderlichen Substanzen in Pufferlösung in das Röhrchen gibt und die gebildete Farbe misst.

Eine weitere Möglichkeit zur Plasmininhibitoren-Entfernung besteht darin, dass man das zu untersuchende Plasma mit Polystyrollatex versetzt, bewegt oder einige Zeit stehen lässt, wobei sich wiederum das Fibrinmonomer an die Oberfläche der Polystyrolphase der Latex bildet, die Latex dann abtrennt und wieder, wie oben beschrieben, als Probe einsetzt.

Für die eigentliche Farbbildungsreaktion inkubiert man während einer zur Ausbildung der Farbe ausreichenden Zeit, beispielsweise 0,5 bis 5 Stunden, und misst dann die gebildete Farbe.

Für die Durchführung des erfindungsgemässen Verfahrens benötigt man Gewebs-Plasminogenaktivator (EPA), der auch als vaskulärer Plasminogenaktivator oder extrinsic-Plasminogenaktivator bezeichnet wird, und Plasminogen. Beide Substanzen sind handelsüblich. Weiter wird ein chromogenes Plasminsubstrat verwendet, beispielsweise ein Plasminsubstrat, welches p-Nitroalin enthält. Bei der Inkubation wird das p-Nitroanilin freigesetzt und lässt sich leicht bei 405 nm messen. Derartige Plasminsubstrate sind bekannt und teilweise auch handelsüblich, wie beispielsweise Tos-Gly-Pro-Lys-p-Nitroanilid und D-Val-Leu-Lys-p-Nitroanilid. Andere geeignete chromogene Plasminsubstrate sind die den angegebenen Nitroaniliden entsprechenden Aminoanilide in Kombination mit einer Kupplungskomponente und einem Oxydationsmittel.

Ein weiterer Gegenstand der Erfindung ist ein Reagenz zur Bestimmung von Fibrinmonomer im Plasma, welches dadurch gekennzeichnet ist, dass es Plasminogen, EPA, chromogenes Plasminsubstrat und Puffer, pH 7,0 bis 9,0 enthält.

Vorzugsweise enthält das erfindungsgemässe Reagenz 0,1 bis 2,0 U/ml Plasminogen, 0,1 bis 10,0 Enheiten Gewebsplasminogenaktivator (EPA), 0,02 bis 2,0 mMol/l chromogenes Plasminsubstrat und 0,01 bis 2,0 Mol/l Puffer.

Zusätzlich kann das erfindungsgemässe Reagenz auch noch oberflächenaktives Mittel, vorzugsweise ein nichtionogenes oberflächenaktives Mittel wie Tween 80® enthalten. Die Konzentration beträgt in diesem Falle vorzugsweise 0,01 bis 1,0 Vol.-%

Als Puffersubstanz kann jede im angegebenen Bereich von 7,0 bis 9,0 puffernde Substanz verwendet werden. Bevorzugt wird Tris-Puffer.

Die Einheiten Plasminogen sind bezogen auf Tos-Gly-Pro-Lys-p-Nitroanilin als Substrat, 37°C und Messung des Plasminogen-Streptokinase-Komplexes. Die oben angegebene Einheit EPA ist definiert als die Menge, welche unter den angegebenen Testbedingungen, aber mit sättigenden Mengen Fibrinmonomer getestet innerhalb von zwei Stunden Inkubationsdauer einen Extinktionsanstieg bei 405 nm von 1,0 ergibt (bei 25°C).

Durch die Erfindung wird dem Fachmann die Möglichkeit gegeben, die Thrombosebereitschaft spezifisch und mit hoher Empfindlichkeit durch ein einfaches Verfahren zu bestimmen.

Das folgende Beispiel erläutert die Erfindung.

Beispiel 1
Reagenz:
Plasminogen 0,8 U/ml
Plasminsubstrat Tos-Gly-Pro-Lys-p-Nitroanilin 0,3 nMol/1,
EPA 2 µg (= ungefähr 1,0 Einheiten)

Tris-HCl 0,07 Mol/l, pH 7,5
Tween 80® 0,07 Vol.-%

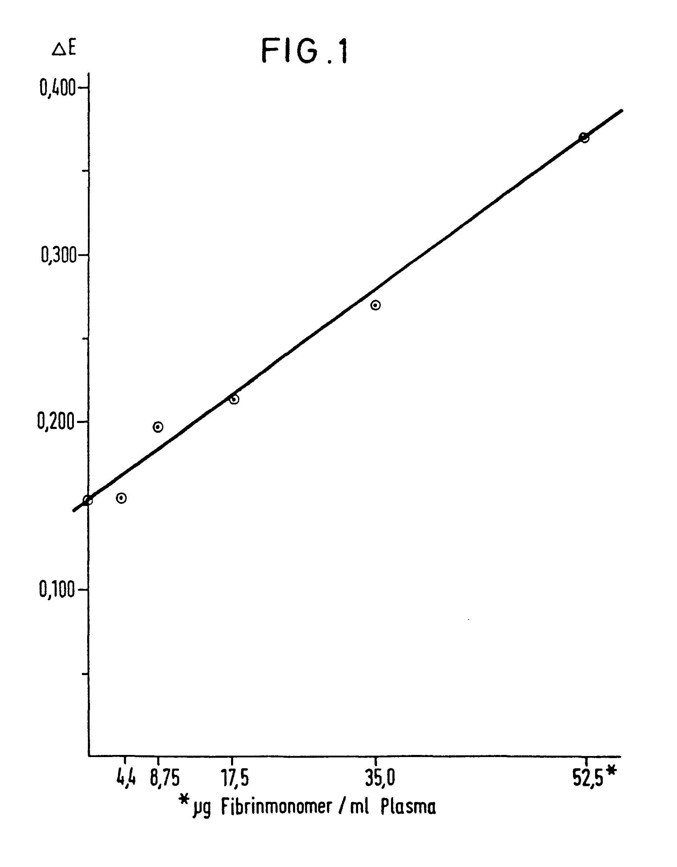

Testdurchführung:
Plasma wurde mit steigenden Mengen Fibrinmonomer versetzt. Je 100 µl Plasma wurden mit 900 µl eiskaltem Wasser und 75 µl Essigsäure 0,25%ig versetzt. Dann wurde 30 Minuten in Eis stehengelassen und anschliessend 10 Minuten bei 3000 U/Min zentrifugiert. Der Niederschlag wurde in 700 µl Puffer gelöst. Je 100 µl Probelösung wurden 900 µl Reagenzlösung zugesetzt. Dann wurde 2 Stunden bei 25°C inkubiert und die Extinktion bei 405 nm gemessen. Die erhaltenen Ergebnisse sind in Fig. 1 der Zeichnung dargestellt. In dieser ist die Extinktion aufgetragen gegen die zugesetzte Menge an Fibrinmonomer. Diese Menge liegt dabei zwischen 0 und 52,5 µg Fibrinmonomer pro ml Plasma.

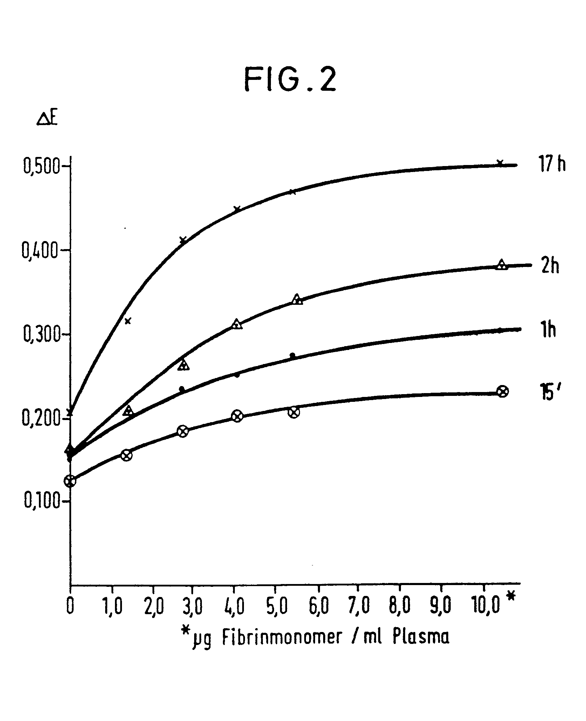

Beispiel 2
Plasma wurde stufenweise mit Fibrinmonomer vesetzt und dann im Verhältnis 1:4 mit Wasser verdünnt. Je 250 µl Plasmaverdünnung wurden in die Bohrungen einer Polystyrol-Mikrotiterplatte gefüllt. Dann wurde bei 4°C stehengelassen, die Probe anschliessend ausgeschüttet und mit Wasser gespült. Danach wurden je 250 µl Reagenz, wie im Beispiel 1, eingefüllt, zwei Stunden bei 25°C inkubiert und anschliessend die Extinktion bei 405 nm gemessen. Die erhaltenen Ergebnisse sind in Fig. 2 der Zeichnung dargestellt. Die Kurvenschar gibt die Ergebnisse bei verschiedener Verweildauer des Plasmas in der Mikrotiterplatte an. Die Verweildauer liegt dabei zwischen 15 Minuten und 17 Stunden.

**Patentansprüche**

1. Verfahren zur Bestimmung von Fibrinmonomer in Plasma, dadurch gekennzeichnet, dass man die Fibrinmonomer enthaltende, von Plasmininhibitoren befreite Plasmaprobe mit Gewebsplasminogenaktivator (EPA), Plasminogen und einem chromogenen Plasminsubstrat in gepufferter Lösung inkubiert und die gebildete Farbe nach Mass für die Fibrinmonomermenge misst.

2. Verfahren nach Anspruch 1, dadurch gekennzeichnet, dass man das zu untersuchende Plasma mit Säure versetzt, den gebildeten Niederschlag abtrennt und in Pufferlösung suspendiert als Probe einsetzt.

3. Verfahren nach Anspruch 2, dadurch gekennzeichnet, dass man das als Säure Essigsäure verwendet.

4. Verfahren nach Anspruch 1, dadurch gekennzeichnet, dass man das zu untersuchende Plasma mit einem Polystyrolkörper inkubiert, danach die Flüssigkeit entfernt und den Polystyrolkörper als Probe einsetzt.

5. Verfahren nach Anspruch 1, dadurch gekennzeichnet, dass man das zu untersuchende Plasma mit Polystyrollatex versetzt, inkubiert, die Latex abtrennt und als Probe einsetzt.

6. Reagenz zur Bestimmung von Fibrinmonomer im Plasma, dadurch gekennzeichnet, dass es Plasminogen, Gewebs-Plasminogenaktivator (EPA), chromogenes Plasminsubstrat und Puffer pH 7,0 bis 9,0 enthält.

7. Reagenz nach Anspruch 6, dadurch gekennzeichnet, dass es 0,1 bis 2,0 U/ml Plasminogen, 0,1 bis 10,0 Einheiten Gewebsplasminogenaktivator, 0,02 bis 2,0 mMol pro Liter chromogenes Plasmainsubstrat und 0,01 bis 0,2 Mol/l Puffersubstanz, pH 7,0 bis 9,0, enthält.

8. Reagenz nach Anspruch 6 oder 7, dadurch gekennzeichnet, dass es ein oberflächenaktives Mittel enthält.

## Claims

1. Process for the determination of fibrin monomer in plasma, characterised in that one incubates the plasma sample containing fibrin monomer and freed from plasmin inhibitors in buffered solution with tissue plasminogen activator (EPA), plasminogen and a chromogenic plasmin substrate and measures the colour formed as measure for the amount of fibrin monomer.

2. Process according to claim 1, characterised in that one mixes plasma to be investigated with acid, separates off the precipitate formed and suspends in buffer solution as sample.

3. Process according to claim 2, characterised in that one uses acetic acid as acid.

4. Process according to claim 1, characterised in that one incubates the plasma to be investigated with a polystyrene body, thereafter removes the liquid and uses the polystyrene body as sample.

5. Process according to claim 1, characterised in that one mixes the plasma to be investigated with polystyrene latex, incubates, separates off the latex and uses as sample.

6. Reagent for the determination of fibrin monomer in plasma, characterised in that it contains plasminogen, tissue plasminogen activator (EPA), chromogenic plasmin substrate and buffer, pH 7,0 to 9,0.

7. Reagent according to claim 6, characterised in that it contains 0,1 to 2,0 U/mol. plasminogen, 0,1 to 10,0 units of tissue plasminogen activator (EPA), 0,02 to 2,0 mMole per litre of chromogenic plasmin substrate and 0,01 to 0,2 Mole/l of buffer substance, pH 7,0 to 9,0.

8. Reagent according to claim 6 or 7, characterised in that it contains a surface-active agent.

## Revendications

1. Procédé de dosage de la fibrine monomère dans le plasma, caractérisé en ce qu'on soumet à l'incubation l'échantillon de plasma contenant la fibrine monomère débarrassé des inhibiteurs de la plasmine, avec de l'activateur de plasmogène tissulaire (EPA), du plasminogène et un substrat de plasmine chromogène en solution tamponnée et mesure la couleur formée en tant que mesure de la quantité de fibrine monomère.

2. Procédé selon la revendication 1, caractérisé en ce qu'on additionne le plasma à d'acide, sépare le précipité formé et l'utilise comme échantillon en suspension dans la solution tampon.

3. Procédé selon la revendication 2, caractérisé en ce qu'on utilise l'acide acétique comme acide.

4. Procédé selon la revendication 1, caractérisé en ce qu'on soumet le plasma à examiner à l'incubation avec un corps en polystyrène, sépare ensuite le liquid et met en œuvre le corps en polystyrène comme échantillon.

5. Procédé selon la revendication 1, caractérisé en ce qu'on additionne le plasma à examiner d'un latex de polystyrène, le soumet à l'incubation, sépare le latex et l'utilise comme échantillon.

6. Réactif pour le dosage de la fibrine monomère dans le plasma, caractérisé en ce qu'il contient du plasminogène, de l'activateur de plasminogène tissulaire (EPA), un substrat de plasmine chromogène et un tampon de pH 7,0 à 9,0.

7. Réactif selon la revendication 6, caractérisé en ce qu'il contient 0,1 à 2,0 U/ml de plasminogène, 0,1 à 10 unités d'activateur de plasminogène tissulaire, 0,02 à 2,0 mmoles par litre de substrat de plasmine chromogène et 0,01 à 0,2 mole/l de substance tampon, pH 7,0 à 9,0.

8. Réactif selon la revendication 6 ou 7, caractérisé en ce qu'il contient un agent tensio-actif.

FIG.1

# FIG.2